Europäisches Patentamt

European Patent Office

Office européen des brevets

(19)

(11) Veröffentlichungsnummer : **0 120 248**
**B1**

(12) **EUROPÄISCHE PATENTSCHRIFT**

(45) Veröffentlichungstag der Patentschrift :
26.04.89

(51) Int. Cl.⁴ : **A 61 K 9/48, A 61 K 9/66**

(21) Anmeldenummer : **84101356.8**

(22) Anmeldetag : **10.02.84**

(54) **Polyethylenglykolhaltige Weichgelatinekapsel und Verfahren zu ihrer Herstellung.**

(30) Priorität : **02.03.83 DE 3307353**

(43) Veröffentlichungstag der Anmeldung :
**03.10.84 Patentblatt 84/40**

(45) Bekanntmachung des Hinweises auf die Patenterteilung : **26.04.89 Patentblatt 89/17**

(84) Benannte Vertragsstaaten :
**AT BE CH DE FR GB IT LI NL SE**

(56) Entgegenhaltungen :
**GB—A— 1 135 709**
**LU—A— 65 929**
**US—A— 2 870 060**
**US—A— 4 198 391**
**Die Akte enthält technische Angaben, die nach dem Eingang der Anmeldung eingereicht wurden und die nicht in dieser Patentschrift enthalten sind.**

(73) Patentinhaber : **R.P. Scherer GmbH**
**Gammelsbacher Strasse 2 Postfach 1243**
**D-6930 Eberbach/Baden (DE)**

(72) Erfinder : **Brox, Werner**
**Kätchen-Paulus-Strasse 6**
**D-6124 Beerfelden (DE)**

(74) Vertreter : **Andrae, Steffen, Dr. et al**
**Patentanwälte Andrae, Flach, Haug, Kneissl Steinstrasse 44**
**D-8000 München 80 (DE)**

EP 0 120 248 B1

**Beschreibung**

Die Erfindung betrifft verbesserte Arzneiformen, und zwar polyethylenglykolhaltige Weichgelatinekapseln, die sich durch eine besonders hohe physikalische Stabilität und eine besonders gute Haltbarkeit auszeichnen.

Nachdem es in den 30iger Jahren gelungen war, Weichgelatinekapseln so herzustellen, daß die Kapselherstellung und -füllung in einem Arbeitsgang erfolgt, haben sich Gelatinekapseln, insbesondere Weichgelatinekapseln als Arzneiform immer stärker durchgesetzt. Sie weisen gegenüber anderen Darreichungsformen eine Reihe von Vorteilen auf. So sind sie geruch- und geschmacklos, lassen sich leicht einnehmen, und dank ihrer Quellfähigkeit und Wasserlöslichkeit werden die Arzneistoffe im Magen leicht freigesetzt. Zahlreiche Arzneistoffe, die sich sonst wegen ihrer Oxidations- und Lichtempfindlichkeit, Thermolabilität oder Hygroskopizität nicht zu anderen Arzneiformen verarbeiten lassen, können ohne Beeinträchtigung ihrer Wirkung verkapselt werden.

Weichgelatinekapseln dienen vorwiegend zur Aufnahme von Flüssigkeiten, und zwar öligen Lösungen, Suspensionen oder Emulsionen. Als Füllmaterialien sind pflanzliche, tierische und mineralische Öle, flüssige Kohlenwasserstoffe, ätherische Öle und auch Polyethylenglykole in Gebrauch. Zur Konsistenzerhöhung werden auch Fette und Wachse verwendet oder zugesetzt.

Polyethylenglykole zeichnen sich dabei gegenüber den anderen möglichen Füllmaterialien für Weichgelatinekapseln durch eine Reihe von Besonderheiten aus. Im Gegensatz zu öligen Flüssigkeiten sind flüssige Polyethylenglykole mit Wasser unbegrenzt mischbar, und auch die festen Polyethylenglykole sind sehr gut wasserlöslich. Da Polyethylenglykole gleichzeitig viele in Wasser schwer- oder gar nicht lösliche Arzneistoffe zu lösen vermögen, ermöglicht die Verwendung von Polyethylenglykolen bei derartigen Arzneistoffen eine besonders vorteilhafte Wirkstofffreisetzung. Schwer wasserlösliche Arzneistoffe, die in Polyethylenglykolen gelöst oder suspendiert und dann in Weichgelatinekapseln abgefüllt sind, zeichnen sich in vielen Fällen durch eine besonders gute Bioverfügbarkeit der Arzneistoffe aus. So ist z. B. in Br. J. clin. Pharmac. (1977), 4, Seite 209 bis 211 berichtet, daß die Bioverfügbarkeit von Digoxin besonders gut ist, wenn der Wirkstoff in Form einer Polyethylenglykollösung, die in eine Weichgelatinekapsel eingekapselt ist, verabreicht wird.

Trotz dieser vor allem biopharmazeutischen Vorteile von Weichgelatinekapseln mit polyethylenglykolhaltigen Füllungen bereitet die Herstellung physikalisch stabiler und haltbarer Kapseln beträchtliche Schwierigkeiten.

Polyethylenglykol besitzt eine hohe Affinität sowohl zu dem Gelatine-Kapselmaterial, als auch zu den in der Kapselhülle verwendeten Weichmachern. Als Weichmacher für Gelatinekapseln mit den üblichen Füllmaterialien wird vor allem Glycerol verwendet, wobei jedoch auch Sorbit und in begrenztem Umfang Polyethylenglykole selbst als Weichmacher bekannt sing (vergl. Czetsch-Lindenwald und Fahrig, Arnzeikapseln, Aulendorf, 1962, Seite 26/27 oder R. Voigt, Lehrbuch der pharmazeutischen Technologie, 3. Auflage, 1979, Seite 244). Dabei geht man allgemein davon aus, daß Glycerol infolge seiner stärkeren Hygroskopizität ein wirksamerer Weichmacher ist als Sorbit, weshalb in den meisten Fällen Glycerol verwendet wird (vergl. auch DE-PS 22 09 526). Infolge der Wechselwirkungen der polyethylenglykolhaltigen Füllungen mit den Weichmacher enthaltenden Weichgelatinekapseln kommt es meist bereits kurz nach der Herstellung derartiger Kapseln zu Veränderungen der Härte und Flexibilität der Kapselhüllen. Die Kapselhüllen werden dabei häufig so spröde, daß sie platzen und die Füllung austritt. Es kommt vor, daß derartige spröde Kapseln bereits bei dem Transport als Bulkware zerstört werden, weil sie den dabei auftretenden mechanischen Belastungen nicht standhalten können.

In anderen Fällen kommt es aufgrund der Affinität von Polyethylenglykolen zu den Kapselhüllen dazu, daß bei der Lagerung Polyethylenglykole aus der Füllung in die Gelatinehülle diffundieren. Da die Polyethylenglykole Weichmachereigenschaften haben, werden die Kapseln sehr weich. Sie kleben aneinander und verformen sich und lassen sich, falls sie in Kunststoffolien eingesiegelt werden, nicht mehr ohne Beschädigung aus der Verpackung herausdrücken. In vielen Fällen diffundiert das Polyethylenglykol durch die Kapselhülle und verschmiert die Kapseloberfläche. Derartige Kapseln müssen als verdorben angesehen werden.

In der DE-PS 22 09 526 sind Weichgelatinekapseln beschrieben, deren Kapselhülle als Weichmacher Glycerol enthält, und deren Füllungen Polyethylenglykole mit einem mittleren Molekulargewicht von 300 bis 600 neben einem geringeren Anteil von Glycerol und Wasser enthalten. Die in dieser Patentschrift konkret beschriebenen Kapseln enthalten dabei das Polyethylenglykol ausschließlich Polyethylenglykol 400. Es wird in der DE-PS 22 09 526 gesagt, daß derartig zusammengesetzte Kapseln optimale Ergebnisse liefern. Versucht man jedoch, die in der DE-PS 22 09 526 angegebenen Kapselzusammensetzungen auf Kapseln mit anderen Wirkstoffen oder anderen Kapselgrößen zu übertragen, so stellt man fest, daß immer wieder Mißerfolge dahingehend auftreten, daß die Kapseln, ihre Härte und Flexibilität während der Lagerung verändern, spröd werden oder erweichen, oder daß Polyethylenglykol durch die Kapselhülle diffundiert. Auch wenn man Kapseln entsprechend der in der DE-PS 22 09 526 beschriebenen Kapselzusammensetzung und mit dem dort angegebenen Wirkstoff herstellt, stellt man fest, daß sich die Härte der Kapseln im Verlauf der Lagerung sehr stark verändert. Es besteht daher weiterhin ein Bedürfnis nach polyethylenglykolhaltigen Weichkapseln, die auf reproduzierbare Weise als stabile und dauerhafte Arzneiformen hergestellt werden können.

Ein flüssiges Polyethylenglykol enthaltende Weichkapseln sind ferner auch der US-A-2 870 060 bekannt. Die dort beschriebenen Weichkapseln enthalten als Kapselfüllgut 5 bis 40 Gew.-% eines Alkalisalzes eines Dialkylsulfosuccinats im Gemisch mit einem flüssigen Polyethylenglykol, vorzugsweise Polyethylenglykol 400. Die Kapselwände enthalten als Weichmacher Sorbit. Die relative Stabilität der beschriebenen Kapseln wird auf ein besonderes synergistisches Zusammenwirken der Füllgutbestandteile Wirkstoff und Polyethylenglykol 400 zurückgeführt.

In der US-A-4 198 391 sind Weichkapseln für die Verabreichung des Wirkstoffs Digitoxin oder Digoxin beschrieben, die den Wirkstoff in einem flüssigen Polyethylenglykol enthalten, wobei ausschließlich Polyethylenglykol 400 durch Beispiele belegt ist. Die Kapselwandungen enthalten ein Weichmachergemisch aus Sorbit und Glycerin. Die Kapseln wurden im Hinblick auf eine rasche Verfügbarkeit der Wirkstoffe formuliert, ohne daß auf eine Langzeitstabilität der Kapseln besonders geachtet wurde.

Der Erfindung liegt die Aufgabe zugrunde, polyethylenglykolhaltige Weichkapseln und ein Verfahren zu deren Herstellung anzugeben, bei denen die Art und die Mengen der neben verschiedenen beliebigen Wirkstoffen als Kapselfüllungen vorhandenen Materialien und die Kapselwandungen so aufeinander abgestimmt sind, daß Weichgelatinekapseln mit polyethylenglykolhaltigen Lösungen oder Suspensionen erhalten werden, bei denen die nach der Herstellung der Kapseln optimal eingestellte Härte und Flexibilität während einer Langzeitlagerung unverändert bleibt, und bei denen kein Polyethylenglykol durch die Kapselhülle diffundiert und die Kapseloberfläche nicht mit Polyethylenglykol verschmiert wird.

Diese Aufgabe wird erfindungsgemäß durch eine Weichgelatinekapsel mit einer Hülle aus Gelatine und einem Weichmacher auf der Basis von Polyhydroxyverbindungen sowie einer Kapselfüllung, die als flüssiges Polyethylenglykol und einen niederen mehrwertigen Alkohol und mindestens einen Wirkstoff enthält, gelöst, die dadurch gekennzeichnet ist, daß

a. die aufgetrocknete Hülle der Kapsel 4 bis 40 Gew.-% Sorbitane oder Sorbit und Sorbitane enthält,
b. das in der Kapselfüllung zur Lösung oder Suspendierung des Wirkstoffs verwendete Polyethylenglykol zu mindestens 50 Gew.-% ein Polyethylenglykol mit einem mittleren Molekulargewicht von 600 ist, und
c. das Kapselfüllgut bis zu 20 Gew.-% Glycerol und/oder 1,2-Propylenglykol enthält.

Nach langwierigen Untersuchungen und Variation einer großen Vielzahl von an sich bekannten Materialen, nämlich verschiedenen Polyethylenglykolen, verschiedenen Zusätzen zu diesen Polyethylenglykolen, verschiedenen Weichmachern und Mengenverhältnissen wurde überraschenderweise gefunden, daß die erfindungsgemäße Kombination von Merkmalen sicherstellt, daß die Weichgelatinekapseln während der Lagerzeit zwischen Produktion und ihrem Verbrauch durch die Patienten eine gleichbleibende Härte aufweisen, und daß keinerlei Polyethylenglykole durch die Kapselhülle diffundieren.

Ein sehr wesentlicher Aspekt der vorliegenden Erfindung liegt in der Auswahl des Polyethylenglykols, da gefunden wurde, daß die erstrebten Eigenschaften nur dann erzielt werden, wenn mindestens 50 Gew.% des verwendeten Polyethylenglykols Polyethylenglykol mit dem mittleren Molekulargewicht von 600 verwendet wird. Besonders gut reproduzierbare Ergebnisse werden erhalten, wenn ausschließlich Polyethylenglykol mit dem mittleren Molekulargewicht von 600 verwendet wird. Polyethylenglykol dieses Molekulargewichts liegt hinsichtlich seiner Eigenschaften an der Grenze zwischen flüssigen und festen Polyethylenglykolen, und ist unter verschiedenen Bezeichnungen im Handel erhältlich.

Es ist für die Lösung der vorliegenden Aufgabe ferner meist wichtig, daß die Kapselfüllung bis zu 20 Gew.-% Glycerol und/oder 1,2-Propylenglykol enthält. Die Reproduzierbarkeit der Ergebnisse ist besonders gut, wenn die Kapselfüllung Glycerol in einer Menge zwischen 5 und 10 Gew.-% enthält.

Üblicherweise liegt der Gehalt an Wirkstoff in der Kapselfüllung zwischen 1 und 7 Gew.%, wobei jedoch ohne weiteres auch andere Wirkstoffanteile möglich sind.

In der Regel enthält die Füllung der getrockneten Weichgelatinekapsel auch noch einen Anteil von Wasser, der zwischen 4 und 20 Gew.% betragen kann. In der Regel liegt der Wassergehalt im Bereich von 6 bis 10 Gew.%, meist in der Größenordnung von 7 oder 8 Gew.-%, wobei dieser Wasseranteil bei der Trockung der wasserhaltigen Gelatinekapseln aufgrund der Affinität der Kapselfüllung zu dem Wasser in die Kapselfüllung gelangt.

Es ist für die vorliegende Erfindung von außerordentlicher Wichtigkeit, daß für die Kapselhülle eine Gelatine verwendet wird, die als Weichmacher mindestens teilweise eine solche Menge an Sorbitanen oder Sorbit und Sorbitanen enthält, daß der Anteil von Sorbitanen oder Sorbit und Sorbitanen in der getrockneten Kapselhülle zwischen 4 und 40 Gew.% liegt. Neben Sorbitanen oder Sorbit und Sorbitanen kann als Weichmacher auch noch ein zusätzlicher Anteil an Glycerol vorhanden sein. Es ist jedoch nicht möglich, die der Erfindung zugrunde liegende Aufgabe zu lösen, wenn man eine Gelatine verwendet, die als Weichmacher ausschließlich Glycerol enthält. Besonders gute und reproduzierbare Ergebnisse werden erhalten, wenn der Gehalt an Sorbit und Sorbitanen, im Bereich von 8 bis 30 Gew.%, insbesondere im Bereich von 10 bis 25 Gew.% liegt. Sorbitane sind Sorbitanhydride, die aus Sorbit durch intramolekulare Abspaltung von Wasser entstehen.

Im Handel sind unter verschiedenen Bezeichnungen auch Mischungen von Sorbit und Sorbitanen erhältlich. Diese Mischungen können, bedingt durch die Herstellungsweise, neben Sorbit und Sorbitanen noch geringe Mengen Mannit, Isosorbid oder andere Polyole enthalten. Sofern der Sorbit- und Sorbitangehalt in dem oben angegebenen Bereich liegt, sind derartige Mischungen aus Sorbit und Sorbitanen als Weichmacher für die Kapselhülle gut geeignet. Ferner kann die Kapselhülle noch andere übliche Hilfsstoffe enthalten, z. B. Konservierungsmittel wie p-Aminobenzoesäureester und Kaliumsorbat oder Farbstoffe und Farbpigmente.

Es war für den Fachmann nicht vorhersehbar, daß das durchschnittliche Molekulargewicht der Polyethylenglykols einen so großen Einfluß auf die physikalische Stabilität von Weichgelatinekapseln hat, insbesondere im Hinblick auf das Diffusionsvermögen von Polyethylenglykolen durch die intakt bleibende Gelatinehülle. Wie die nachfolgenden Vergleichsversuche zeigen, ist nämlich das z. B. in der DE-PS 22 09 526 als besonders vorteilhaft hervorgehobene Polyethylenglykol 400 als Füllmaterial für die Herstellung von Weichgelatinekapseln nicht geeignet, die den hohen Anforderungen gemäß der Aufgabe der vorliegenden Erfindung gerecht werden. Es ist ferner als überraschend anzusehen, daß in Verbindung mit der angegebenen besonderen Kapselfüllung Sorbit und Sorbitane bessere Weichmachereigenschaften aufweisen, als das in dieser Hinsicht üblicherweise überlegene Glycerol. Es war ebenfalls nicht absehbar, daß der teilweise oder vollständige Ersatz des üblichen Weichmachers Glycerol in der Kapselhülle durch Sorbitane oder Sorbit und Sorbitane eine Aufrechterhaltung der gewünschten eingestellten Härte der Kapsel während der Lagerung ermöglicht.

Die erfindungsgemäß zusammengesetzten Weichgelatinekapseln zeichnen sich ferner dadurch aus, daß aufgrund der optimalen Abstimmung von Kapselfüllung und Kapselmaterial kaum ein Einfluß der verschiedenen Wirkstoffe im Kapselfüllgut feststellbar ist. Die erfindungsgemäßen Weichkapseln können daher führ sehr viele verschiedenartige Arzneistoffe verwendet werden, die in Polyethylenglykolen löslich oder suspendierbar sind, und bei denen sich biopharmazeutische Vorteile, insbesondere im Bereich der Wirkstofffreisetzung ergeben, wenn sie mit Polyethylenglykolen zusammen verabreicht werden. Kapseln ausgezeichneter physikalischer Stabilität wurden beispielsweise mit den Arzneistoffen Diazepam, Indometacin und Dipyridamol erhalten.

Wird der Wirkstoff in dem polyethylenglykolhaltigen Füllgut nicht gelöst sondern suspendiert, so kann zum Schutz der Suspension vor einer unerwünschten Sedimentation des Wirkstoffes während des Herstellungsprozesses eine Viskositätserhöhung des Füllgutes mit Hilfe fester Polyethylenglykole erforderlich sein. Der Zusatz von bis zu 20 Gew.% Polyethylenglykolen mit einem mittleren Molekulargewicht über 600 hat keinerlei negativen Einfluß auf die physikalische Stabilität der auf diese Weise hergestellten Kapseln.

Die erfindungsgemäßen Weichgelatinekapseln können abgesehen von den erfindungsgemäß besonders ausgewählten Füll- und Kapselmaterialien auf übliche Weise, beispielsweise nach dem bekannten Scherer-Verfahren, hergestellt werden. Wie allgemein bekannt ist, wird bei der Herstellung von Weichgelatinekapseln in einem Arbeitsgang zunächst eine feuchte Kapsel erzeugt, die anschließend aufgetrocknet wird. Demgemäß enthält die zur Herstellung der Kapselhülle verwendete Gelatine zum Zeitpunkt der Herstellung einen beträchtlichen Anteil an Wasser, der beispielsweise in der Größenordnung von 30-40 Gew.% liegen kann. Das Füllmaterial kann dagegen völlig wasserfrei eingesetzt werden. Beim Auftrocknen der Kapseln kommt es dann in der Regel dazu, daß das in der Kapselwandung der feuchten Kapsel enthaltene Wasser nicht nur verdampft, sondern zu einem geringen Teil auch in die Kapselfüllung übergeht. Gemäß der vorliegenden Erfindung wirken sich derartige Wasseranteile im Füllungsgut nicht negativ auf die Kapselstabilität und Haltbarkeit aus. Gemäß der vorliegenden Erfindung ist es für die an sich übliche Herstellung der erfindungsgemäßen Weichgelatinekapseln nur wichtig, darauf zu achten, daß der Gehalt an Sorbit und Sorbitanen nach der Trocknung im oben angegebenen Bereich liegt. Der Wassergehalt der bei der Herstellung verwendeten Gelatine muß daher entsprechend berücksichtigt werden.

Nachfolgend wird die Erfindung anhand eines Ausführungsbeispiels und zweier Vergleichsbeispiele näher erläutert.

## Beispiele

Bei dem Beispiel und den Vergleichsbeispielen wurde einheitlich so vorgegangen, daß unter identischen Bedingungen Weichgelatinekapseln der angegebenen Zusammensetzungen hergestellt wurden, die für die Prüfung ihrer Haltbarkeit in feuchtigkeitsdichten Glasflaschen bei 20 °C aufbewahrt wurden. Die physikalische Stabilität wurde mit Hilfe der beiden folgenden Meßverfahren überprüft :

1. Bestimmung des in die Hülle diffundierten Gehalts an Polyethylenglykolen

Die zu untersuchende Kapsel wurde nach der angegebenen Lagerzeit aufgeschnitten, und das polyethylenglykolhaltige Füllgut wurde mit einem organischen Lösungsmittel ausgewaschen. Anschließend wurde die Kapselhülle aufgelöst, die darin enthaltenen Polyethylenglykole wurden silyliert und anschließend auf gaschromatographischem Wege quantitativ analysiert.

2. Messung der Kapselhärte

Die Messung der Kapselhärte erfolgte mit einem handelsüblichen Härtetester, bei dem die zu prüfende Kapsel zwischen einer sich langsam aufwärtsbewegenden Platte und einem Meßfühler innerhalb von 20 Sekunden um 2 mm zusammengedrückt wird. Die von der Kapsel ausgeübte Gegenkraft wird am Anzeigegerät in Newton abgelesen. Unter diesen Prüfbedingungen weisen Härtewerte über 11 N auf eine unzureichende Flexibilität der Kapseln hin. Werte unter 9 N werden für zu weiche Kapseln erhalten.

## Beispiel (gemäß der Erfindung)

Unter Verwendung einer üblichen Maschine zur Herstellung von Weichgelatinekapseln wurden aus den folgenden Ausgangsmaterialen feuchte Kapseln hergestellt :

Material für die Kapselhülle

| | |
|---|---|
| Gelatine | 168,0 mg |
| Glycerol 85 %ig | 52,0 mg |
| Sorbit und Sorbitane | 43,0 mg |
| Wasser | 137,0 mg |

Material für die Kapselfüllung

| | |
|---|---|
| Polyethylenglykol 600 | 459,0 mg |
| Glycerol 85 %ig | 51,0 mg |
| Diazepam (Wirkstoff) | 5,0 mg |

Die erhaltenen feuchten Kapseln wurden getrocknet, und es wurden trockene Kapseln der folgenden Zusammensetzung erhalten :

Hülle

| | |
|---|---|
| Gelatine | 168,0 mg |
| Glycerol 85 %ig | 52,0 mg |
| Sorbit und Sorbitane | 43,0 mg |

Füllung

| | |
|---|---|
| Polyethylenglykol 600 | 459,0 mg |
| Glycerol 85 %ig | 51,0 mg |
| Wasser | 38,0 mg |
| Diazepam | 5,0 mg |

## Vergleichsbeispiel 1

In diesem Vergleichsbeispiel werden Kapseln untersucht, bei denen die Hülle kein Sorbit und Sorbitane enthielt, während als Füllmaterial das gemäß der vorliegenden Erfindung auszuwählende Polyethylenglykol 600 verwendet wird.

Unter identischen Bedingungen wie in Beispiel 1 wurden feuchte Kapseln der folgenden Zusammensetzung hergestellt :

Material für die Hülle

| | |
|---|---|
| Gelatine | 175,0 mg |
| Glycerol 85 %ig | 102,0 mg |
| Wasser | 123,0 mg |

Material für die Füllung

| | |
|---|---|
| Polyethylenglykol 600 | 459,0 mg |
| Glycerol 85 %ig | 51,0 mg |
| Diazepam | 5,0 mg |

Die getrockneten Kapseln wiesen die folgende Zusammensetzung auf :

Hülle

| | |
|---|---|
| Gelatine | 175,0 mg |
| Glycerol 85 %ig | 102,0 mg |

Füllung

| | |
|---|---|
| Polyethylenglykol 600 | 459,0 mg |
| Glycerol 85 %ig | 51,0 mg |
| Wasser | 35,0 mg |
| Diazepam | 5,0 mg |

Vergleichsbeispiel 2

Bei diesem Vergleichsbeispiel wurde in der Kapsel Sorbit und Sorbitane neben Glycerol als Weichmacher verwendet, während für die Kapselfüllung Polyethylenglykol 400 verwendet wurde. Die Zusammensetzung der wie in Beispiel 1 hergestellten feuchten Kapsel war:

Material für die Hülle

| | |
|---|---|
| Gelatine | 168,0 mg |
| Glycerol 85 %ig | 52,0 mg |
| Sorbit und Sorbitane | 43,0 mg |
| Wasser | 137,0 mg |

Material der Füllung

| | |
|---|---|
| Polyethylenglykol 400 | 459,0 mg |
| Glycerol 85 %ig | 51,0 mg |
| Diazepam | 5,0 mg |

Die aus diesen feuchten Kapseln erhaltenen getrockneten Kapseln wiesen die folgende Zusammensetzung auf:

Hülle

| | |
|---|---|
| Gelatine | 168,0 mg |
| Glycerol 85 %ig | 52,0 mg |
| Sorbit und Sorbitane | 43,0 mg |

Füllung

| | |
|---|---|
| Polyethylenglykol 400 | 459,0 mg |
| Glycerol 85 %ig | 51,0 mg |
| Wasser | 43,0 mg |
| Diazepam | 5,0 mg |

Ergebnisse der Stabilitätstests

1. Messung der Kapselhärte

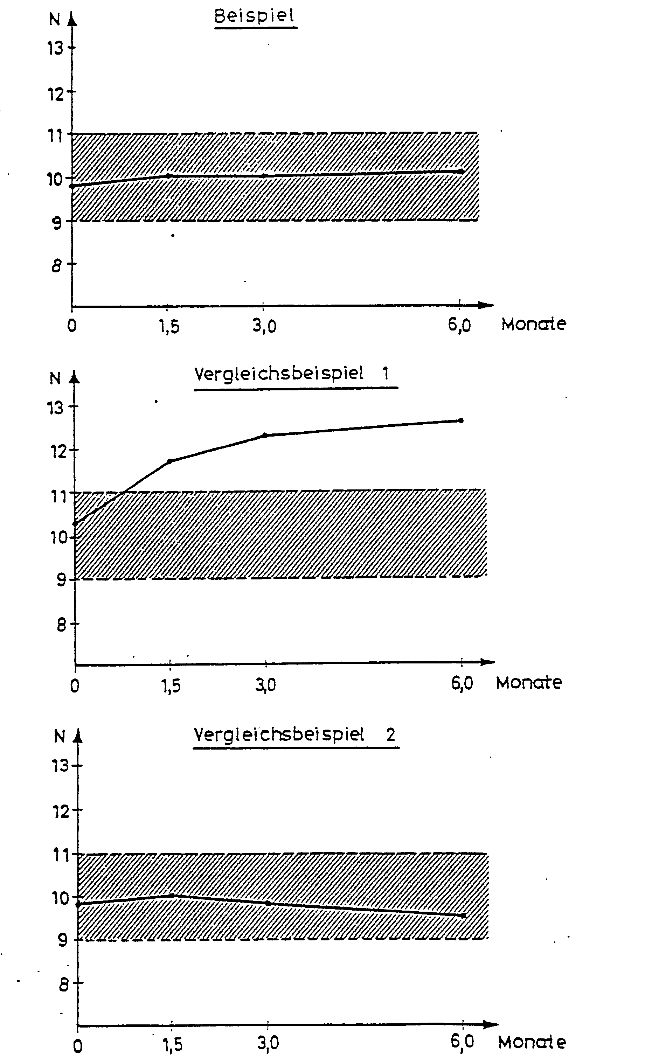

Die Kapseln des Beispiels sowei der beiden Vergleichsbeispiele wurden nach der Herstellung sowie nach einer Lagerzeit von 1 1/2, 3 und 6 Monaten in ihrer Härte überprüft. Die Ergebnisse der Härtmessungen sind in der folgenden Tabelle 1 sowie in Fig. 1 wiedergegeben.

Tabelle I

| Kapseln gemäß | Kapselhärte (N) nach einer Lagerzeit von | | | |
|---|---|---|---|---|
| | O | 1,5 | 3,0 | 6,0 Monaten |
| Beispiel | 9,8 | 10,0 | 10,0 | 10,1 |
| Vergleichsbeisp. 1 | 10,3 | 11,7 | 12,3 | 12,6 |
| Vergleichsbeisp. 2 | 9,8 | 10,0 | 9,8 | 9,5 |

Aus den Meßwerten, die in der beiliegenden Zeichnung aufgetragen sind, geht hervor, daß die Kapseln des Beispiels mit Polyethylenglykol 600 in der Füllung und Sorbit und Sorbitane in der Hülle ihre optimale Härte von ca. 10 N über den gesamten Zeitraum von 6 Monaten nicht veränderten.

Die Kapseln des Vergleichsbeispiels 1, welche in der Kapselhülle kein Sorbit und keine Sorbitane enthielten, veränderten ihre Härte sehr rasch und wurden spröde.

Die Kapseln des Vergleichsbeispiels 2, die in der Füllung kein Polyethylenglykol 600, sondern Polyethylenglykol 400 enthielten, wurden nach 3 bzw. nach 6 Monaten etwas weicher. Das Weicherwerden der Kapseln deutet darauf hin, daß sich der Weichmachergehalt der Kapselhülle während der Lagerzeit erhöht hat, was durch die nachfolgenden Untersuchungsergebnisse des Polyethylenglykolgehaltes der Kapselhülle bestätigt wird.

2. Bestimmung des in die Hülle diffundierten Gehalts an Polyethylenglykolen

Die Kapseln des Beispiels sowie des Vergleichsbeispiels 2 wurden nach 3 und 6 Monaten Lagerung auf ihren Polyethylenglykolgehalt in der Kapselhülle untersucht. Die Ergebnisse sind in der folgenden Tabelle 2 wiedergegeben.

Tabelle 2

| Kapseln gemäß | Gehalt an Polyethylenglykol in der Hülle nach einer Lagerzeit von | |
| --- | --- | --- |
| | 3,0 | 6,0 Monaten |
| Beispiel | 1,7 mg | 3,6 mg |
| Vergleichsbeisp. 2 | 9,5 mg | 15,5 mg |

Aus den Meßwerten geht hervor, daß bei den Kapseln des Beispiels nur eine sehr geringe Menge an Polyethylenglykol 600 in die Kapselhülle diffundiert, während im gleichen Zeitraum bei den Kapseln des Vergleichsbeispiels 2 die Diffusion von Polyethylenglykol 400 in die Kapselhülle erheblich stärker ist.

Aus dem Beispiel und den beiden Vergleichsbeispielen ergibt sich, daß nur die gleichzeitige entsprechende Wahl des Weichmachers und des bestimmten Polyethylenglykols Kapseln mit den gewünschten hervorragenden Eigenschaften liefert. Es ist dabei darauf hinzuweisen, daß die Vergleichsversuche bereits im Hinblick auf die Lehre der vorliegenden Erfindung durchgeführt wurden, während sich im Stand der Technik nicht einmal konkrete Hinweise finden, die auf die Vorteile einer Verwendung von Polyethylenglykol 600 oder von Sorbitanen oder Sorbit und Sorbitanen als Weichmacher in derartigen Weichgelatinekapseln hindeuten. Ungezielt durchgeführte Versuche mit anderen bekannten Polyethylenglykolen und Kapseln, die als Weichmacher nur Glycerol enthielten, lieferten noch schlechtere Ergebnisse als die Vergleichsversuche.

Gemäß der vorliegenden Erfindung wird es somit möglich, Weichgelatinekapseln hervorragender Haltbarkeit und Beständigkeit herzustellen, wobei zwar für sich genommen bekannte, wenn auch zum Teil in anderen Zusammenhängen verwendete Bestandteile verwendet werden, die bisher noch nicht in der angegebenen Form kombiniert wurden.

**Patentansprüche**

1. Weichgelatinekapsel mit einer Hülle aus Gelatine und einem Weichmacher auf der Basis von Polyhydroxyverbindungen sowie einer Kapselfüllung, die ein flüssiges Polyethylenglykol und einen niederen mehrwertigen Alkohol und mindestens einen Wirkstoff enthält, dadurch gekennzeichnet, daß

a. die aufgetrocknete Hülle der Kapsel 4 bis 40 Gew.-% Sorbitane oder Sorbit und Sorbitane enthält,

b. das in der Kapselfüllung zur Lösung oder Suspendierung des Wirkstoffs verwendete Polyethylenglykol zu mindestens 50 Gew.-% ein Polyethylenglykol mit einem mittleren Molekulargewicht von 600 ist, und

c. das Kapselfüllgut bis zu 20 Gew.-% Glycerol und/oder 1,2-Propylenglykol enthält.

2. Weichgelatinekapsel nach Anspruch 1, dadurch gekennzeichnet, daß die Kapselfüllung der getrockneten Kapsel zusätzlich zwischen 4 bis 20 Gew.% Wasser enthält.

3. Weichgelatinekapsel nach Anspruch 1 oder 2, dadurch gekennzeichnet, daß die Kapselfüllung als Polyethylenglykol ausschließlich Polyethylenglykol mit einem mittleren Molekulargewicht von 600 enthält.

4. Weichgelatinekapseln nach Anspruch 1 oder 2, dadurch gekennzeichnet, daß das in der Kapselfüllung verwendete Polyethylenglykol zu mindestens 80 Gew.% ein Polyethylenglykol mit einem mittleren Molekulargewicht von 600 und bis zu 20 Gew.% ein Polyethylenglykol mit einem mittleren Molekulargewicht über 600 ist.

5. Verfahren zur Herstellung einer Weichgelatinekapsel nach einem der Ansprüche 1 bis 4, dadurch gekennzeichnet, daß man in an sich bekannter Weise eine Kapselfüllung, die sich aus einem Wirkstoff, einem Polyethylenglykol, das mindestens 50 Gew.-% eines Polyethylenglykols mit einem mittleren

7

Molekulargewicht von 600 enthält, und bis zu 20 Gew.-% Glycerol und/oder 1,2-Propylenglykol zusammensetzt, in eine wasserhaltige Gelatinehülle einkapselt, die einen Anteil von Sorbitanen oder Sorbit und Sorbitanen enthält, und daß man die erhaltene Kapsel trocknet, so daß die aufgetrocknete Kapselhülle 4 bis 40 Gew.-% Sorbitane oder Sorbit und Sorbitane enthält.

## Claims

1. A soft gelatin capsule comprising a shell of gelatin and a softener on the basis of polyhydroxy compounds, and a capsule filling comprising a liquid polyethylene glycol and a low polyhydric alcohol and at least one active substance, characterized in that
   a. the dried shell of the capsule contains 4 to 40 % by weight sorbitanes or sorbitol and sorbitanes,
   b. at least 50 % by weight of the polyethylene glycol used in the filling for dissolving or suspending the active substance is a polyethylene glycol having a mean molecular weight of 600, and
   c. the capsule filling comprises up to 20 % by weight of glycerol and/or 1,2-propylene glycol.

2. A soft gelatin capsule in accordance with claim 1, characterized in that the capsule filling of the dried capsule contains additionally between 4 and 20 % by weight of water.

3. A soft gelatin capsule in accordance with claim 1 or 2, characterized in that the polyethylene glycol of the capsule filling consists exclusively of polyethylene glycol having a mean molecular weight of 600.

4. A soft gelatin capsule in accordance with claim 1 or 2, characterized in that of the polyethylene glycol used in the capsule filling, a portion of at least 80 % by weight is a polyethylene glycol having a mean molecular weight of 600 and a portion of up to 20 % by weight is polyethylene glycol having a mean molecular weight of above 600.

5. A method for producing a soft gelatin capsule in accordance with any one of claims 1 to 4, characterized in that a capsule filling made up of active substance, a polyethylene glycol containing at least 50 % by weight of a polyethylene glycol having a mean molecular weight of 600 and up to 20 % by weight of glycerol and/or 1,2-propylene glycol is conventionally enclosed in an aqueous gelatin shell containing an amount of sorbitanes or sorbitol and sorbitanes and that the capsule so obtained is dried so that the dried capsule shell contains 4 to 40 % by weight of sorbitanes or sorbitol and sorbitanes.

## Revendications

1. Gélule molle comportant une enveloppe de gélatine et un plastifiant à base de composés polyhydroxyle, ainsi qu'une charge contenant un polyéthylène-glycol liquide et un alcool polyvalent inférieur, de même qu'au moins une substance active, caractérisée en ce que :
   a. l'enveloppe séchée de la gélule contient 4 à 40 % en poids de sorbitannes ou de sorbitol et de sorbitannes,
   b. le polyéthylène-glycol utilisé dans la charge de la gélule pour la dissolution ou la mise en suspension de la substance active est constitué, pour au moins 50 % en poids, d'un polyéthylène-glycol ayant un poids moléculaire moyen de 600, et
   c. la matière de charge de la gélule contient jusqu'à 20 % en poids de glycérol et/ou de 1,2-propylène-glycol.

2. Gélule molle selon la revendication 1, caractérisée en ce que la charge de la gélule séchée contient, en outre, entre 4 et 20 % en poids d'eau.

3. Gélule molle selon la revendication 1 ou 2, caractérisée en ce que sa charge contient, comme polyéthylène-glycol, exclusivement du polyéthylène-glycol d'un poids moléculaire moyen de 600.

4. Gélules molles selon la revendication 1 ou 2, caractérisées en ce que le polyéthylène-glycol utilisé dans la charge de ces gélules est constitué, pour au moins 80 % en poids, d'un polyéthylèneglycol ayant un poids moléculaire moyen de 600, et d'une fraction de polyéthylène-glycol d'un poids moléculaire supérieur à 600 allant jusqu'à 20 % en poids.

5. Procédé de fabrication d'une gélule molle selon une des revendications 1 à 4, caractérisé en ce qu'on encapsule, de façon connue en soi, dans une enveloppe de gélatine contenant de l'eau, une charge de gélule constituée d'une substance active, d'un polyéthylène-glycol contenant au moins 50 % en poids d'un polyéthylène-glycol ayant un poids moléculaire moyen de 600, et d'une fraction de glycérol et/ou de 1,2-propylène-glycol allant jusqu'à 200 % en poids, cette enveloppe de gélatine contenant une certaine quantité de sorbitannes ou de sorbitol et de sorbitannes, et en ce qu'on sèche la gélule obtenue de telle sorte que son enveloppe séchée contienne 4 à 40 % en poids de sorbitannes ou de sorbitol et de sorbitannes.

Beispiel

Vergleichsbeispiel 1

Vergleichsbeispiel 2